# EUROPEAN PATENT APPLICATION

(11) **EP 2 159 280 A1**
(43) Date of publication of application: **03.03.2010**
(21) Application number: 07743410.8
(22) Date of filing: 15.05.2007
(51) Int. Cl.: C12N 15/09

(54) **REPLICATION/TRANSCRIPTION SYSTEM FOR INFLUENZA VIRUS GENOME USING YEAST CELL**

(71) Applicant: University of Tsukuba, Tsukuba-shi, Ibaraki 305-8577 (JP)
(72) Inventor: NAGATA, Kyosuke, Tsukuba-shi Ibaraki 305-8577 (JP); NAITO, Tadasuke, Tsukuba-shi Ibaraki 305-8577 (JP)
(74) Representative: Albutt, Jodie
(86) International application number: PCT/JP2007/059975
(87) International publication number: WO 2008/139627

(57) **Abstract**

There have been no studies which introduced a negative-strand RNA virus genome in yeast cells or which identified that such genome was replicated in yeast cells. The present inventors have successfully developed a system in which vRNP purified from influenza virus particles is introduced into yeast (*Saccharomyces cerevisiae*) and the transcription and replication from the viral RNA genome are supported by the yeast. The virus genome replication, the virus gene transcription and the virus protein expression occurring in yeast cells in which a great amount of genetic information is accumulated will permit identifying specific host cell factors that are involved in influenza virus infection, influenza virus growth in host and pathogenicity. The present invention thus provides a tool and a technique that are essential for the development of anti-influenza virus agents.

SEQUENCE LISTING

<110> University of Tsukuba
<120> Replication and transcription system for influenza virus genome using yeast
cell
<130>
<140> PCT/JP2007/059975
<141> 2007-05-15
<160> 52


<210> 1
<211> 32
<212> DNA
<213> Yeast
<220>
<221> Promoter
<223> Chromosome IV SUB2 up-FOR

<400> 1
gttaggtacc gaatacttgg ccaagatctt gg


<210> 2
<211> 31
<212> DNA
<213> Yeast
<220>
<221> Promoter
<223> Chromosome IV SUB2 up-REV

<400> 2
acttcccggg tccaacaggc aaacagttcc g


<210> 3
<211> 32
<212> DNA
<213> Yeast
<220>

<221> Terminator
<223> Chromosome IV SUB2 down-FOR

<400> 3
ccgtgaattc aacgtagtgt agtcatttcg tg

<210> 4
<211> 32
<212> DNA
<213> Yeast
<220>
<221> Terminator
<223> Chromosome IV SUB2 down-REV

<400> 4
cgttgaattc ttctccaaca tcgatatcag ag

<210> 5
<211> 30
<212> DNA
<213> Yeast
<220>
<221> Primer
<223> ACT1 cDNA ACT1-FOR

<400> 5
atggattctg aggttgctgc tttggttatt

<210> 6
<211> 20
<212> DNA
<213> Yeast
<220>
<221> Primer
<223> ACT1 cDNA ACT1-REV

<400> 6

gcgtgaggta gagagaaacc

<210> 7
<211> 26
<212> DNA
<213> Yeast
<220>
<221> Primer
<223> ADH1 cDNA ADH1-FOR

<400> 7
atgtctatcc cagaaactca aaaagg

<210> 8
<211> 22
<212> DNA
<213> Yeast
<220>
<221> Primer
<223> ADH1 cDNA ADH1-REV

<400> 8
aacgttttca cccatgccga ca

<210> 9
<211> 18
<212> DNA
<213> Yeast
<220>
<221> Primer
<223> RDN25-1 cDNA RDN25-1-FOR

<400> 9
ctggtacctt cggtgccc

<210> 10

<211> 19
<212> DNA
<213> Yeast
<220>
<221> Primer
<223> RDN25-1 cDNA RDN25-1-REV

<400> 10
cgctatcggt ctctcgcca


<210> 11
<211> 56
<212> DNA
<213> Yeast
<220>
<221> Primer
<223> SUB2 cDNA SUB2-FOR (His-)

<400> 11
tttcatatgc atcatcatca tcatcatggt tcacacgaag gtgaagaaga
tttatt


<210> 12
<211> 24
<212> DNA
<213> Yeast
<220>
<221> Primer
<223> SUB2 cDNA SUB2 1062-FOR

<400> 12
gctcgttaca aggctttcaa agat


<210> 13
<211> 44
<212> DNA
<213> Yeast

<220>
<221>  Primer
<223>  SUB2 cDNA  B2-REV

<400>  13
ccggatcctt  aattattcaa  ataagtggac  ggatcaatgc  cttc


<210>  14
<211>  43
<212>  DNA
<213>  Yeast
<220>
<221>  Primer
<223> TRP1 cDNA TRP1-FOR

<400>  14
ggacggctag  caacgacatt  actatatata  taatatagga  agc


<210>  15
<211>  48
<212>  DNA
<213>  Yeast
<220>
<221>  Primer
<223>  TRP1 cDNA TRP1-REV

<400>  15
ggccgcgtct  cccttgggcc  cggcaagtgc  acaaacaata  cttaaata


<210>  16
<211>  61
<212>  DNA
<213>  Yeast
<220>
<221>  Terminator
<223>  CYC1 TT-FOR
<400>  16

ccccaagctt agatctgcgc ccgggtcacc atcatgtaat tagttatgtc
acgcttacat t

<210> 17
<211> 48
<212> DNA
<213> Yeast
<220>
<221> Terminator
<223> CYC1 TT-REV

<400> 17
cggactagtg atatcccggg gtctagagca aattaaagcc ttcgagcg

<210> 18
<211> 35
<212> DNA
<213> Influenza virus
<220>
<221> Primer
<223> Segment 3 vRNA PA-FOR

<400> 18
gatcccgggc atatggaaga ttttgtgcga caatg

<210> 19
<211> 22
<212> DNA
<213> Influenza virus
<220>
<221> Primer
<223> Segment 3 vRNA PA 664-FOR

<400> 19
atcacaggaa caatgcgcaa gc

<210> 20
<211> 26
<212> DNA
<213> Influenza virus
<220>
<221> Primer
<223> Segment 3 vRNA PA 800-REV

<400> 20
ggttcaattc tagcatttac ttcttt


<210> 21
<211> 26
<212> DNA
<213> Influenza virus
<220>
<221> Primer
<223> Segment 3 cRNA PA 3' -END

<400> 21
agtagaaaca aggtactttt ttggac


<210> 22
<211> 24
<212> DNA
<213> Influenza virus
<220>
<221> Primer
<223> Segment 3 c/mRNA PA 1356-FOR

<400> 22
cacattgcaa gcatgagaag gaat


<210> 23
<211> 23
<212> DNA
<213> Influenza virus

<220>
<221> Primer
<223> Segment 3 c/mRNA PA 1683-REV

<400> 23
ggcacttctt agaagcatat ctc


<210> 24
<211> 32
<212> DNA
<213> Influenza virus
<220>
<221> Primer
<223> Segment 5 vRNA NP-FOR

<400> 24
ggaattcata tggcgtctca aggcaccaaa cg


<210> 25
<211> 21
<212> DNA
<213> Influenza virus
<220>
<221> Primer
<223> Segment 5 vRNA NP 444-FOR

<400> 25
gacgatgcaa cggctggtct g


<210> 26
<211> 20
<212> DNA
<213> Influenza virus
<220>
<221> Primer
<223> Segment 5 vRNA NP 614-REV

<400> 26
agcattgttc caactccttt

<210> 27
<211> 26
<212> DNA
<213> Influenza virus
<220>
<221> Primer
<223> Segment 5 cRNA NP 3' -END

<400> 27
agtagaaaca agggtatttt tcttta

<210> 28
<211> 23
<212> DNA
<213> Influenza virus
<220>
<221> Primer
<223> Segment 5 c/mRNA NP 846-FOR

<400> 28
cggtctgcac tcatattgag agg

<210> 29
<211> 18
<212> DNA
<213> Influenza virus
<220>
<221> Primer
<223> Segment 5 c/mRNA NP 1121-REV

<400> 29
gaaagcttcc ctcttggg

<210> 30
<211> 26
<212> DNA
<213> Influenza virus
<220>
<221> Primer
<223> Segment 7 cRNA M1 3' -END

<400> 30
agtagaaaca aggtagtttt ttactc

<210> 31
<211> 20
<212> DNA
<213> Influenza virus
<220>
<221> Primer
<223> Segment 7 c/mRNA M1 897-FOR

<400> 31
taaatacgga ctgaaaggag

<210> 32
<211> 26
<212> DNA
<213> Influenza virus
<220>
<221> Primer
<223> Segment 7 c/mRNA M1 1007-REV

<400> 32
ttactccagc tctatgctga caaaat

<210> 33
<211> 48
<212> DNA
<213> Influenza virus

<220>
<221> Primer
<223> PB1 cDNA PB1-FOR 1

<400> 33
ccccaagctt gatatcgcgg ccgccaccat ggatgtcaat ccgacctt

<210> 34
<211> 37
<212> DNA
<213> Influenza virus
<220>
<221> Primer
<223> PB1 cDNA PB1-REV 1

<400> 34
cgcgtcgacg gtacctattt ttgccgtctg agctctt

<210> 35
<211> 53
<212> DNA
<213> Influenza virus
<220>
<221> Primer
<223> PB2 cDNA PB2-FOR 1

<400> 35
cgcggatccc gggcggccgc caccatggaa agaataaaag
aactaagaaa tct

<210> 36
<211> 33
<212> DNA
<213> Influenza virus
<220>
<221> Primer
<223> PB2 cDNA PB2-REV 1

<400> 36
cgcgctcgag ctaattgatg gccatccgaa ttc

<210> 37
<211> 48
<212> DNA
<213> Influenza virus
<220>
<221> Primer
<223> PA cDNA PA-FOR

<400> 37
cgcggatccg cggccgccac catggaagat tttgtgcgac aatgcttc

<210> 38
<211> 34
<212> DNA
<213> Influenza virus
<220>
<221> Primer
<223> PA cDNA PA-REV

<400> 38
gcggggccct aactcaatgc atgtgtaagg aagg

<210> 39
<211> 38
<212> DNA
<213> Influenza virus
<220>
<221> Primer
<223> NP cDNA NP-FOR

<400> 39
ttggatccaa aatggctact aaaggtacta aaagatct

<210> 40
<211> 36
<212> DNA
<213> Influenza virus
<220>
<221> Primer
<223> NP cDNA NP-REV

<400> 40
ggaattcatc ttaattgtcg tactcctctg cattgt


<210> 41
<211> 34
<212> DNA
<213> Influenza virus
<220>
<221> Primer
<223> PB1 cDNA PB1-FOR 2

<400> 41
gatcccgggc atatggatgt caatccgacc ttac


<210> 42
<211> 37
<212> DNA
<213> Influenza virus
<220>
<221> Primer
<223> PB1 cDNA PB1-REV 2

<400> 42
gcgcctcgag ctactaatcg atgcttgcta gcatttc


<210> 43
<211> 31
<212> DNA

<213> Influenza virus
<220>
<221> Primer
<223> PB2 cDNA PB2-FOR 2

<400> 43
gcgcctcgag cgcgagatac tcacaaaaac c

<210> 44
<211> 37
<212> DNA
<213> Influenza virus
<220>
<221> Primer
<223> PB2 cDNA PB2-REV 2

<400> 44
gcgcctcgag ctactagctt tgatcaacat catcatt

<210> 45
<211> 40
<212> DNA
<213> Influenza virus
<220>
<221> Primer
<223> Segment 5 vRNA Seg 5 digestion

<400> 45
ggaattcata tggtgatgga attggtcaga atgatcaaac

<210> 46
<211> 56
<212> DNA
<213> Influenza virus
<220>
<221> Primer
<223> yEGFP cDNA NS yEGFP-FOR

<400> 46
gtagcaaaag cagggtgaca aagacataat gtctaaaggt gagagattat
tcactg

<210> 47
<211> 50
<212> DNA
<213> Influenza virus
<220>
<221> Primer
<223> yEGFP cDNA NS yEGFP-REV

<400> 47
agtagaaaca agggtgtttt ttttatttgt acaattcatc cataccatgg

<210> 48
<211> 37
<212> DNA
<213> Influenza virus
<220>
<221> Primer
<223> Segment 8 vRNA T7 vNS-FOR

<400> 48
taatacgact cactataagt agaaacaagg gtgtttt

<210> 49
<211> 22
<212> DNA
<213> Influenza virus
<220>
<221> Primer
<223> Segment 8 vRNA vNS-REV

<400> 49
agtagaaaca agggtgtttt tt

<210> 50
<211> 37
<212> DNA
<213> Influenza virus
<220>
<221> Primer
<223> Segment 8 cRNA T7 cNS-FOR

<400> 50
taatacgact cactataagc aaaagcaggg tgacaaa

<210> 51
<211> 20
<212> DNA
<213> Influenza virus
<220>
<221> Primer
<223> Segment 8 cRNA cNS-REV

<400> 51
agcaaaagca gggtgacaaa

<210> 52
<211> 53
<212> RNA
<213> Influenza virus
<220>
<221> Primer
<223> Segment 8 vRNA 53-merVwt

<400> 52
aguagaaaca aggguguuuu uucauaucau uuaaacuuca
cccugcuuuu gcu

## Description

### TECHNICAL FIELD

The present invention relates to influenza virus genome replication and transcription systems in yeast cells. In more detail, the invention relates to systems in which the transcription and replication of negative-strand RNA genome of influenza virus are expressed in yeast cells..

### BACKGROUND ART

Influenza viruses infect animals including humans and birds. Viruses grow by entering host animals and infecting host cells. After entry into the host cells, the viruses grow utilizing functions of viral factors and of various host factors including subcellular organelles. To understand the mechanism of virus genome replication or the molecular mechanism of pathogenicity, it is critical to identify and analyze host factors involved in these mechanisms.

Easy introduction of DNA virus genome into cells permitted detailed analysis of the molecular mechanism of replication and transcription of DNA virus, and a large number of host factors have been found to be involved in the mechanism. The analysis of these host factors also led to findings in the replication and transcription mechanisms of host cell genomes, and contributed to the development of biology. A study has found that RNA viruses having a positive-strand RNA genome are infectious as the RNA genome alone (Non-patent Document 1). Accordingly, if an RNA virus genome synthesized *in vitro* is introduced into cells, transcription and replication occur from the virus genome. Similar to the DNA viruses, the virus growth process of positive-strand RNA virus was successfully analyzed by introducing reconstituted virus RNA genome into cells. The analysis has revealed the molecular mechanisms of transcription and replication, and has identified relevant host factors (Non-patent Documents 2, 3 and 4).

However, RNA viruses including influenza viruses that have a negative-strand RNA genome do not show infectivity even when the RNA genome alone is introduced into cells. The transcription and replication from negative-strand RNA genomes require a virus-derived,̅ RNA-dependent RNA polymerase. The RNA genome becomes infectious by forming a viral RNA-protein complex (vRNP: viral RNA-viral RNA polymerase-nucleocapsid protein complex) with the virus polymerase. Accordingly, for the reconstituted RNA genome to function in the cell, the genome should be introduced as vRNP or viral factors such as virus polymerase required for transcription and replication should be expressed (Non-patent Documents 5 and 6). This inconvenience has delayed the development of studies on negative-strand RNA viruses in cells using reconstituted RNA genomes.

Influenza virus reverse-genetics systems were established recently, and the virus genome transcription and replication as well as pathogenicity have been studied extensively (Non-patent Document 7). However, most of such studies target viral factors, and few focus on functions of host factors. With the current systems, even identifying functional host factors is difficult. New systems for identifying unknown host factors involved in influenza virus growth are desired.
[Non-patent Document 1]
   Neumann G, et al., J Gen Virol, Vol. 83, pp. 2635-2662 (2002)
[Non-patent Document 2]
   Kushner D. B., et al., Proc Natl Acad Sci USA, Vol. 100, pp. 15764-15769 (2003)
[Non-patent Document 3]
   Panavas T, et al., Proc Natl Acad Sci USA, Vol. 102, pp. 7326-7331 (2005)
[Non-patent Document 4]
   Ahlquist P, et al., J Virol, Vol. 77, pp. 8181-8186 (2003)
[Non-patent Document 5]
   Luytjes W, et al., Cell, Vol. 59, pp. 1107-1113 (1989)
[Non-patent Document 6]
   Yamanaka K, et al., Proc Natl Acad Sci USA, Vol. 88, pp. 5369-5373 (1991)
[Non-patent Document 7]
   Neumann G, et al., Proc Natl Acad Sci USA, Vol. 96, pp. 9345-9350 (1990)

### SUMMARY OF THE INVENTION

### TECHNICAL PROBLEM

There have been no studies which identified that viruses such as influenza viruses having a negative-strand RNA genome replicated their genomes in yeast cells. The virus genome replication, the virus gene transcription and the virus protein expression occurring in yeast cells in which a great amount of genetic information is accumulated will permit identifying specific host cell factors that are involved in influenza virus infection, influenza virus growth in host and pathogenicity, and will provide a tool and a technique that are essential for the development of anti-influenza virus agents.

### SOLUTION TO THE PROBLEM

The present inventors have successfully developed the world's first technique in which vRNP purified from influenza virus particles is introduced into yeast (*Saccharomyces cerevisiae*) and the transcription and replication of viral RNA genome are supported in the yeast. The constitution of the present invention as means for providing such technique is described below.

In an aspect of the present invention, a method for preparing a negative-strand virus genome replication system in yeast comprises introducing into yeast cells a viral RNA genome-viral protein complex (vRNP) purified from negative-strand RNA virus particles, and replicating and transcribing the negative-strand RNA genome in the yeast cells.

The negative-strand RNA virus is preferably influenza virus.

The vRNP is introduced by being mixed with a yeast spheroplast in the presence of polyethylene glycol.

In another aspect of the invention, a method for preparing an influenza virus genome replication system comprises introducing into yeast cells an influenza viral RNA genome incorporated with a foreign reporter gene, and replicating and expressing the gene in the yeast cells.

A kit according to the present invention is a kit for preparing an influenza virus genome replication system, which kit comprises at least yeast cells, a purified influenza virus vRNP, reagents for preparing a yeast cell spheroplast, transfection reagents, and a vector for expressing influenza virus protein.

A screening method according to the present invention comprises screening viral factors of negative-strand RNA virus and host factors using a negative-strand virus genome replication system in yeast that is prepared by the foregoing method.

An aspect of the invention is directed to a method for screening host factors involved in the replication and transcription of influenza virus genome using an influenza virus genome replication system in yeast that is prepared by the foregoing method.

Another aspect of the invention is directed to a method for screening anti-influenza virus agents which targets at replication and transcription of virus genome using an influenza virus genome replication system in yeast that is prepared by the foregoing method. A further aspect of the invention is directed to a method for screening anti-virus agents which targets at interaction between a viral factor and a host factor using an Influenza virus genome replication system in yeast that is prepared by the foregoing method.

Another aspect of the invention provides a kit for measuring the expression level of a foreign reporter gene incorporated in an influenza viral RNA genome to determine functions of an anti-influenza virus candidate affecting the replication or transcription activity of influenza virus genome in yeast, the interaction between a viral factor and a host factor, or the virus growth-promoting effects of host factors.

Another aspect of the invention is a yeast transformant in which yeast is transformed by introduction of a negative-strand RNA virus genome.

### ADVANTAGEOUS EFFECTS OF THE INVENTION

Yeasts are model eukaryotic cells and are genetically analyzable. Therefore, mutant strains promoting or inhibiting virus genome replication are obtained easily from the yeasts. And comprehensive identification of host factors will be possible. Virus genome replication systems in yeast have been established for positive-strand RNA viruses such as brome mosaic virus^{20,21}, and a large number of viral factors and host factors⁶ have been identified. In contrast, there have been no in-yeast virus genome replication systems established for negative-strand RNA viruses. The present inventors here show the possibility of such a replication system for influenza virus.

If the system is developed to constitute a system in which the transcription or replication activity of influenza virus is measured in yeast cells by measuring the expression level of a reporter gene, a screening technique for new anti-virus agents will be possible targeting at viral polymerases responsible for virus genome replication. Further, with the system, influences of mutation in viral protein on transcription and replication will be easily detected. Various applications of the system are expected.

### BRIEF DESCRIPTION OF THE DRAWINGS

Fig. 1 shows replication and transcription of influenza virus genome in yeast cells. "Overlay" means overlaid panels. "Seg. 5" and "Seg. 3" represent segment 5 and segment 3 of viral RNA, respectively.
Fig. 2 shows complementation of segment 5-deletion vRNP. "RNase H digestion" means digestion by RNase H.
Fig. 3 shows replication and transcription of model viral RNA.
Fig. 4 shows effects of SUB2 deletion. The word "mock" means imitation. "Seg. 7" means segment 7.

### DETAILED DESCRIPTION OF THE INVENTION

The terminologies and expressions used in the present specification are only explanatory and are not restrictive. The gene engineering terminologies such as "transfection or transfect", "viral factors", "host factors, "positive-strand" and "negative-strand" have the same definitions as understood by the person skilled in the art.

By the words that viral genomes are "infectious"⁶⁻⁹, it is meant that viral genomes (including engineered genomes) are active in their replication and transcription when the viral genomes are introduced into cells.
Virus replication requires virus-derived factors, and is totally dependent on host cell functions and machineries. Identification of host factors involved in host functions and machineries is critical to reveal the molecular mechanism of virus replication and pathogenicity. Such researches have been advanced for DNA viruses. In the case of positive-strand RNA viruses, the RNA genome is "infectious", and researches thereof have been advanced⁶⁻⁹. In contrast, the genome RNA of negative-strand RNA viruses is not infectious, although vRNP complexes isolated from virions are infectious¹⁰. Because of the non-infectivity, the replication from negative-strand RNA genome is not readily expressed in cells and studies have been delayed.

Influenza virus has an eight-segmented single negative-strand RNA genome. The influenza viral RNA genome binds to RNA-dependent RNA polymerase composed of PB1, PB2 and PA subunits and nucleoprotein (NP) (these are "viral factors") and thereby forms a viral RNA-protein complex (vRNP)¹¹. The vRNP is a basic unit necessary for transcription and replication of virus genome.
In the method for preparing a negative-strand RNA virus genome replication system in yeast, a viral RNA-viral protein complex (vRNP) purified from negative-strand RNA virus particles is introduced into yeast cells, and the negative-strand RNA genome is transcribed and replicated in the yeast cells.

The method is detailed in PREFERRED EMBODIMENTS FOR CARRYING OUT THE INVENTION. The use of the negative-strand RNA virus genome replication system in yeast established in the invention will advance researches on identification, as well as clarification of functions, of host cell factors involved in virus growth and pathogenicity by expression of virus genome. The advanced researches will lead to the development of effective anti-virus agents.

The negative-strand RNA virus is preferably influenza virus. Influenza viruses have rapid genetic transformation, and newly emerge or reemerge frequently. Due to these facts, it is difficult to produce effective influenza vaccines today. It is therefore desired that influenza virus genome manipulating techniques that support the development of influenza vaccines and anti-influenza virus agents should be established early.

The method of the invention is applicable to other negative-strand RNA viruses such as paramyxoviruses, coronaviruses, filoviruses, arenaviruses and rhabdoviruses.
Host cells may be animal or plant cells (for example, cultured cells). However, some cell systems, in particular cultured mammal cells, have limited genetic techniques. In contrast, yeast cells are suitable since they are cultured easily and have a far faster cell growth rate than animal cells. Yeast cells are easily available as various kinds of strains including mutants and subspecies, and their characteristics are already revealed in detail. Genomic analysis has been completed for yeasts that are regarded as model eukaryotic cell organisms. Their base sequences are known, so that the yeasts can be genetically analyzed based on the genetic information. Mutant strains promoting or inhibiting viral genome replication are easily obtained, and comprehensive identification of host factors is possible. With the above advantages, the systematic and comprehensive screening indicated by characters expressed in the yeast cell system is far more greatly advantageous than other cell systems from the viewpoints of labor and costs.

The yeasts used in the invention are not particularly limited. Various yeast mutant strains may be used as required. In particular, *Saccharomyces cerevisiae* which is easily obtained and has known characteristics is suitable.

vRNP may be introduced into yeast cells by a method known as transfection introducing foreign genes into cells. Exemplary methods are lithium acetate methods and electroporation methods. In a preferred method, vRNP purified from virions is mixed with a yeast spheroplast in the presence of polyethylene glycol.

In an aspect of the invention, a method for preparing an influenza virus genome replication system comprises introducing into yeast cells an influenza viral RNA genome incorporated with a foreign reporter gene, and replicating and expressing the gene in the yeast cells. In the method, a model virus genome is introduced into yeast cells wherein the model virus genome is an influenza virus genome in which a gene expression essential part remains but an open reading frame is replaced with a reporter gene. The method provides a system which supports the replication of the model virus genome and the transcription and expression of the reporter gene.

The reporter genes may be genes which permit measurement of the activity of virus genome replication or the transcription control activity. To this end, genes that are foreign to yeast genome and permit easy measurement of expression are selected.

Reporter gene may be used to obtain yeast mutant strains affecting the virus genome replication activity among comprehensively mutated yeast cells based on the activity of the reporter gene. By determining the mutation causing genes by complementary test, the host factors involved in the virus genome replication can be identified.

Suitable reporter genes for newly introducing characters as the indication include luciferase genes, yeast auxotrophic marker genes (*URA3, LYS2, TRP1, HIS3, LUE2*) and drug resistance genes (neomycin resistance genes, zeocin resistance genes). The open reading frame of viral genome may be replaced with the reporter gene by a known method.
A kit according to the present invention is a kit for preparing an influenza virus genome replication system. It comprises at least yeast cells, a purified influenza virus vRNP, reagents for preparing yeast cell spheroplast, transfection reagents, and a vector for expressing influenza virus protein.

The kit includes materials required to carry out the method of the invention and a manual listing recommended protocols. In detail, the kit may include reagents for preparing yeast cell spheroplast, transfection reagents and/or primers and reagents for gene amplification.

In another embodiment, the kit may include other reagents or materials as required. For example, reagents for isolating influenza virus vRNP from virions, microtiter plates and PCR tools may be included as kit components. The reagents include primers, enzymes, buffers, wash fluids and lysates.

The kit according to the present invention eliminates the complicate handling of a large number of reagents and devices, and allows for rapid and simple processing and analysis.
In another aspect of the invention, a screening method comprises screening for interaction between a negative-strand RNA viral factor and a host factor with use of the negative-strand RNA virus genome replication system in yeast that is prepared by the foregoing method. Compounds targeting at the interaction between host factors and viral factors can affect viral replication and can be powerful candidates for anti-virus agents. A method for screening for such compounds forms another aspect of the invention.
Once influenza virus has infected host cells, its genome RNA (negative-strand) is transcribed to a complementary positive-strand, and a virus protein is produced using the synthesized mRNA. The host cells do not contain any enzymes transcribing the genome RNA, or any viral RNA-dependent RNA polymerases. The enzymes are encoded in the viral genome and present in virus particles by binding to genome RNA. When cells are infected with the virus, the enzymes enter the cells together with the virus genome and transcribe immediately after the infection. From the negative-strand RNA genome, negative-strand RNA of progeny virus is synthesized via a perfectly complementary positive-strand RNA (cRNA). The RNA polymerases are also involved in this replication process. In spite of the great variety of cellular RNA in cells, only the viral RNA is transcribed and replicated in infected cells. This fact suggests that the viral RNA polymerases must distinguish cellular RNA and viral RNA. The viral RNA-dependent RNA polymerases play a central role in the replication and transcription of viral genomes. For appropriate replication and transcription, however, the polymerases probably need help of nuclei of infected cells where these reactions take place or of host factors present in cellular cytoplasm.

A study using SUB2-deletion mutant strain that is a yeast homolog of RAF-2p48, a host factor known to simulate transcription and replication of influenza virus genome, showed that the viral genome replication was lowered. This result suggested that host factors affected an influenza virus replication system in yeast.

Various host factors probably help the virus growth in infected cells, and further involvement of host factors in infection specificity and pathogenicity is assumed.

Research and identification of host factors involved in viral genome functions, particularly in the case of influenza virus, will benefit from a method for screening host factors involved in the viral genome replication and transcription with use of an influenza virus genome replication system in yeast prepared by the foregoing method. The identification of such host factors and analysis of functions thereof are expected to greatly contribute to the development of improved anti-virus agents or next-generation anti-virus agents based on new concepts.
The development of anti-influenza virus agents will benefit from a screening method targeted at replication and transcription of influenza virus genomes using an influenza virus genome replication system in yeast prepared by the foregoing method. In detail, a system is constituted in which the activity of influenza virus genome transcription and replication is measured in yeast based on the expression level of a reporter gene. Such system will enable a screening technique for new anti-virus agents targeted at RNA-dependent RNA polymerases responsible for virus genome replication. In an embodiment, a screening method for anti-influenza virus agents may be targeted at interaction between viral factors and host factors. The present invention establishes an influenza virus genome replication system in yeast cells. With this system and based on genetic information of yeast, the screening method can be performed comprehensively and easily. Compared to conventional screening techniques using cultured cells, the screening methods of the invention are more simple and effective and save costs.
In another aspect of the invention, there is provided a kit for measuring the expression level of a foreign reporter gene incorporated in an influenza viral RNA genome to determine functions of an anti-influenza virus candidate affecting the replication or transcription activity of influenza virus genome in yeast, the interaction between viral factors and host factors, or the virus growth-promoting effects of host factors. The kit is useful in performing the above-described screening methods. The kit includes a set of materials, reagents and protocols required for the screening.

Another aspect of the invention is a yeast transformant in which yeast is transformed by introduction of a negative-strand RNA virus genome in yeast cells. The negative-strand RNA virus genomes may be influenza virus genomes, or negative-strand RNA genomes incorporated with foreign reporter genes.

### PREFERRED EMBODIMENTS FOR CARRYING OUT THE INVENTION

### Method 1

### - Yeast strains and introduction of DNA, RNA and vRNP into yeast cells

Yeast strain YPH499 (*MATa*, *ura3-52, lys2-801, ade2*-*101*, *TRP1-63, his3-200, leu2-1*) was used in all experiments. The *SUB2* deletion strain was generated by replacement of the entire *SUB2* open reading frame with *TRP1* (*SUB2D::TRP1* fragment). A lithium acetate-polyethylene glycol method was used to transform yeast cells with plasmid DNAs. Introduction of RNA and vRNP was performed according to the procedure described for transformation of RNA with minor modifications (see Method 2).

### - Total RNA extraction and RT-PCR

Extraction of total RNA from yeast cells was performed as described in Method 2. For detection of influenza virus RNAs (vRNA, cRNA and mRNA), cellular mRNAs (*ACT1*, *ADH1* and *SUB2*) and rRNA (*RDN25-1*), cDNAs were synthesized using reverse transcriptase (TOYOBO) and specific primers. The sequences of all the primers used in this study are summarized in Tables 1 to 3. cDNAs were then subjected to PCR using Taq DNA Polymerase (NEB) and sets of primers. Amplified double-stranded DNAs were separated through 7% polyacrylamide gel electrophoresis and visualized by ethidium bromide.

### - Indirect immunofluorescence assay

Indirect immunofluorescence assay of yeast cells with anti-NP antibody was performed as described in literature²². DNA was visualized by DAPI staining.

### - Preparation of vRNP complexes and vRNP complexes devoid of segment 5 vRNA

In this specification, all viral resources were derived from influenza A/Puerto Rico/8/34 (PR/8) virus. The purification of virions and the isolation of vRNP complexes therefrom were performed as already described²³. To prepare vRNP devoid of segment 5 vRNA, 10 ml of vRNA (10 ml containing 1 mg of NP) was incubated at 37 °C for 5 minutes in the presence of 0.6 ml of 5 M NaCl and 100 ng of oligonucleotides (Segment 5 digestion: from Table 5) complementary to a part of segment 5 vRNA. Then, the mixture was diluted by addition of 40 ml of 12.5 mM Tris-HCl (pH 7.9) (containing 5 mM MgCl₂ and 1.25 mM dithiothreitol). Thirty units of RNase H (TaKaRa) was added, and the reaction mixture was incubated at 37°C for 5 minutes. The oligonucleotides in the reaction were completely digested with 4 U of RQ DNase I (Invitrogen) in the presence of 25 U of RNase inhibitor (TOYOBO) at 37°C for 10 minutes.

### - In vitro RNA synthesis with T7 RNA polymerase

To construct a DNA template for *in vitro* transcription by T7 RNA polymerase, DNA fragments containing yEGFP (yeast enhanced green fluorescent protein, green fluorescent protein that was modified to codon optimized for gene expression in yeast) gene (sandwiched by 5' - and 3'-terminal sequences of influenza A virus (PR/8) segment 8 vRNA encoding non-structural protein (NS)) were amplified by PCR with primers, NS-yEGFP-FOR and NS-yEGFP-REV using pRGO1 (encoding yEGFP) as template. The amplified fragment was designated NS-yEGFP DNA. For addition of the T7 promoter sequence, overlap extension was performed by PCR with primer sets, either T7 vNS-FOR and yNS-REV, or T7 cNS-FOR and cNS-REV using the NS-yEGFP DNA fragments as template. The vNS-yEGFP and cNS-yEGFP RNAs were synthesized in a transcription reaction containing T7 RNA polymerase (TaKaRa) and the amplified DNA fragments.

### - Western blotting analysis

Exponentially growing yeast cells were collected by low-speed centrifugation and washed twice with 0.9% NaCl. The cells were suspended in a buffer (50 mM Tris-HCl (pH 7.4), 5 mM MgCl₂, 1 M sorbitol, and 3 mM dithiothreitol) containing 100 mg/ml Zymolyase 100T (SEIKAGAKU CORPORATION). The cells were incubated at 30°C for 60 minutes with occasional mixing. The spheroplasts were collected by low-speed centrifugation, resuspended in a cell lysis buffer (50 mM Tris-HCl (pH 7.4), 150 mM NaCl, 0.1% NP-40, and 7 M urea), and lysed by sonication. The cell lysates were cleared by centrifugation and subjected to separation through 7.5% SDS-polyacrylamide gel electrophoresis (SDS-PAGE) followed by western blotting with anti-virus protein antibodies (Preparation of antibodies are described in Method 2).

### - In vitro influenza virus RNA synthesis

*In vitro* influenza virus RNA synthesis was performed as described in literature¹³. Briefly, RNA synthesis was carried out at 37°C for 60 minutes in a final volume of 25 ml containing 50 mM HEPES-NaOH (pH 7.9), 3 mM MgCl₂, 50 mM KCl, 1.5 mM dithiothreitol, 500 mM each ATP, CTP and UTP, 25 mM GTP, 5 mCi of [a-³²P] GTP (3,000 Ci/mmol), 4 U of RNase inhibitor, 250 mM ApG dinucleotide, 10 ng of a 53-nucleotide-long negative-strand model RNA template (53 merVwt: from Table 5), and vRNP (containing 10 ng of NP) as an enzyme source. RNA products were subjected to separation through 10% PAGE in the presence of urea (8 M), and visualized by autoradiography.

### Method 2

### - Yeast strains and culture conditions

Yeast strain YPH499 (*MATa, ura3-52, lys2-801*, *ade2-101*, *TRP1-63, his3-200, leu2-1*) was used in all experiments. Yeast cells were grown at 30°C in yeast extract-peptone-dextrose medium containing adenine (YPDA). For protein expression induced by galactose, single colonies were first inoculated into synthetic defined (SD) medium containing 2% glucose. Yeast cells were grown at 30°C until the optical density at 610 nm (OD₆₁₀) reached 0.6-0.8. Then, the yeast cells were collected by centrifugation at 800 x g and washed twice with phosphate-buffered saline (PBS). The collected yeast cells were cultured in synthetic galactose (SG) medium containing 2% galactose for 6 to 12 hours, allowing the induced expression of proteins.

The following plasmids were generated for construction of a *SUB2* deletion strain. The sequences of all oligonucleotides and primers used in this study are summarized in Tables 1 to 3. The SUB2 deletion strain was generated by replacement of the entire SUB2 open reading frame with the *TRP1* (*SUB2D::TRP1* fragment) (Lorenz *et al.*). Promoter and terminator fragments of the *SUB2* gene were amplified by PCR with primers SUB2 up-FOR and SUB2 up-REV for the upstream fragment, and SUB2 down-FOR and SUB2 down-REV for the downstream fragment, using the yeast genomic DNA as template. The upstream fragment was digested with *Kpn*I and *Sma*I, and cloned into KpnI- and *Sma*I-digested pBluescript (pBS) plasmid. The resultant plasmid was designated pBS-SUB2-upstream. The downstream fragment was phosphorylated with T4 polynucleotide kinase (TOYOBO), and cloned into pBS-SUB2-upstream plasmid which had been digested with *Bam*HI and blunted with Klenow fragment (TaKaRa). The generated plasmid, designated pBS-SUB2, contained a unique *Sma*I site between the promoter and the terminator fragments.

*TRP1* gene fragment was excised from pRS314 plasmid by digestion with *Eco*O109 I and SspI. To construct a plasmid for disruption of SUB2 (pBS-SUB2-*TRP1*), the excised *TRP1* gene fragment was blunted with Klenow fragment and cloned into *Sma* I-digested pBS-SUB2 plasmid.

The SUB2D: : *TRP1* fragment was excised from pBS-SUB2-*TRP1* by digestion with *Bgl*II and *Ban*III, and then used to transform yeast strain YPH499. *TRP1* transformants were tested for the stability of the *TRP1* phenotype. *SUB2* deletion was confirmed by RT-PCR and genomic PCR.

### - Introduction of DNA, RNA and vRNP into yeast cells

A lithium acetate-polyethylene glycol method was used to transform yeast cells with plasmid DNAs (Ito *et al*.). Introduction of RNA and vRNP was performed according to the procedure described for transformation of RNA with minor modifications (Russell *et al.*).

Yeast cells were grown at 30°C in 50 ml of a complete medium (YPDA: yeast extract-peptone-dextrose medium containing adenine) to a cell density of 3 x 10⁷ cells/ml. The yeast cells were collected by centrifugation (at 800 x g for 2 minutes), and the supernatant was removed. The cells were washed with 20 ml of a 0.9% NaCl solution and then with 20 ml of a 1 M sorbitol solution, and resuspended in 20 ml of a SCEM solution (1 M sorbitol, 0.1 M sodium citrate (pH 5.8), 10 mM EDTA, and 30 mM 2-mercaptoethanol). One hundred twenty-five units of Zymolyase-100T was added to the suspension containing the yeast cells, and the cells were incubated at 30°C with occasional mixing. The spheroplasts were collected by low-speed centrifugation, washed with 20 ml of a 1 M sorbitol solution, and then resuspended in 20 ml of a STC solution (1 M sorbitol, 10 mM Tris-HCl (pH 7.4), and 10 mM CaCl₂). The spheroplasts were collected by centrifugation and resuspended in 2 ml of a STC solution. For introduction of RNA or vRNP into the spheroplasts, the spheroplasts (100 ml) were mixed with vRNP or NS-yEGFP RNAs. The mixture was incubated at room temperature for 10 minutes, and 1 ml of a PEG solution (20% polyethylene glycol 6000, 10 mM Tris-HCl (pH 7.4), and 10 mM CaCl₂) was added. The mixture was mixed gently and was allowed to stand at room temperature for 10 minutes. The spheroplasts were collected by centrifugation, and the supernatant was removed. The spheroplasts were resuspended in 1 ml of a SOS solution (1 M sorbitol, 6.5 mM CaCl₂, 50% YPDA or 50% SD).

### - Total RNA extraction from yeast cells, and RT-PCR and genomic PCR

Extraction of total RNA from yeast cells was performed as previously described (Leeds *et al*.). cDNA for detection of segment 5 vRNA was synthesized by reverse transcriptase (TOYOBO) with NP-FOR as primer. This single-stranded cDNA was then PCR-amplified with two specific primers, NP 444-FOR and NP 614-REV.

cDNA for detection of segment 5 cRNA was synthesized by reverse transcriptase with NP 3'-END as primer. The single-stranded cDNA was then PCR-amplified with two specific primers, NP 846-FOR and NP 1121-REV.

cDNA for detection of segment 5 mRNA was synthesized by reverse transcriptase with oligo-dT as primer. The single-stranded cDNA was then PCR-amplified with two specific primers, NP 846-FOR and NP 1121-REV.

cDNA for detection of segment 3 vRNA was synthesized by reverse transcriptase with PA-FOR as primer. The single-stranded cDNA was then PCR-amplifled with two specific primers, PA 664-FOR and PA 800-REV.

cDNA for detection of segment 3 cRNA was synthesized by reverse transcriptase with PA 3'-END as primer. The single-stranded cDNA was then PCR-amplified with two specific primers, PA 1356-FOR and PA 1683-REV.

cDNA for detection of segment 3 mRNA was synthesized by reverse transcriptase with oligo-dT as primer. The single-stranded cDNA was then PCR-amplified with two specific primers, PA 1356-FOR and PA 1683-REV.

cDNA for detection of segment 7 cRNA was synthesized by reverse transcriptase with M1 3'-END as primer. The single-stranded cDNA was then PCR-amplified with two specific primers, M1 897-FOR and M1 1007-REV.

For detection of cellular mRNAs, cDNAs were first synthesized by reverse transcriptase with oligo-dT as primer. For the *ACT1* mRNA of yeast, sense primer ACT1-FOR and anti-sense primer ACT1-REV were used for PCR amplification.

For the *ADH1* mRNA, sense primer ADH1-FOR and anti-sense primer ADH1-REV were used for PCR amplification. For the *SUB2* mRNA, sense primer SUB2 1062-FOR and anti-sense primer SUB2-REV were used for PCR.

For *RDN25-1* rRNA, sense primer RDN25-1-FOR and anti-sense primer RDN25-1-REV were used for PCR.

For detection of *TRP1* gene in yeast genome, *TRP1* fragment was amplified by PCR using *TRP1*-FOR and *TRP1*-REV as primers and the yeast genomic DNA as template. The amplified double-stranded DNAs were subjected to 7% polyacrylamide gel electrophoresis and were visualized by ethidium bromide.

### - Construction of yeast vectors for expression of influenza viral proteins

The following plasmids were constructed for expression of influenza virus RNA polymerase subunits and NP in yeast cells.

DNA fragments containing the CYC1 TT (transcription terminator) sequence were amplified by PCR using CYC1 TT-FOR and CYC1 TT-REV as primers and pYES2 as template. The PCR products were digested with *Hin*dIII and *Spe*I, and cloned into *Hind*III- and *Xba*I-digested pYES2 plasmid. The resultant plasmid, pYES2-CYC1 TT, contained unique *Hin*dIII, *Bgl*II, *Sma*I and *BstE*II sites between the GAL-1 promoter and the CYC1 TT.

To construct pYES2-PB1-CYC1 TT, DNA fragments corresponding to the PB1 coding sequence were amplified by PCR using PB1-FOR and PB1-REV 2 as primers and pcDNA-PB1 (Neumann *et al.*) as template. The PCR products were phosphorylated with T4 polynucleotide kinase and digested with HindIII, and cloned into *Sma*I- and *Hin*dIII-digested pYES2-CYC1 TT.

To construct pYES2-PB2-CYC1 TT, DNA fragments corresponding to the PB2 coding sequence were amplified by PCR using PB2-FOR 1 and PB2-REV 2 as primers and pcDNA-PB2 (Neumann *et al*.) as template. The PCR products were phosphorylated with T4 polynucleotide kinase and digested with *Bam*HI, and cloned into *Sma*I- and *Bgl*I-digested pYES2-CYC1 TT.

To construct pYES2-PA-CYC1 TT, DNA fragments corresponding to the PA coding sequence were amplified by PCR using PA-FOR and PA-REV as primers and pcDNA-PA (Neumann *et al*.) as template. The PCR products were phosphorylated with T4 polynucleotide kinase and digested with *Bam*HI, and cloned into *Sma*I- and *Bgl*I-digested pYES2-CYC1 TT.

To construct plasmids for expression of PB1, PB2 and PA (designated pRS514-PB1, pRS317-PB2 and pRS315-PA, respectively), DNA fragments containing GAL-1 promoter-PB1-CYC1 TT, GAL-1 promoter-PB2-CYC1 TT and GAL-1 promoter-PA-CYC1 TT were excised with SpeI and *Xba*I from pYES2-PB1-CYC1 TT, pYES2-PB2-CYC1 TT and pYES2-PA-CYC1 TT, and cloned into the XbaI site of pRS514, pRS317 and pRS315, respectively.

To construct a plasmid for expression of NP (designated pYES2-NP), DNA fragments corresponding to the NP coding sequence were amplified by PCR using NP-FOR and NP-REV as primers and pCAGGS-NP (Turan *et al.*) as template. The PCR products were digested with *Bam*HI and *Eco*RI, and cloned into *Bam*HI- and *Eco*RI-digested pYES2 plasmid.

### - Antibodies

Rabbit polyclonal antibodies against PB1, PB2 and PA were generated by immunization of 2-month-old female rabbit (Japan White; Tokyo Jikken Doubutsu) with 250 mg each of hexahistidine-tagged (His-) PB1 fragment, His-PB2 fragment and His-PA fragment, respectively, in Freund's complete adjuvant (Sigma).

The His-PB1 fragment with a hexahistidine tag at its amino terminus (a mutant of PB1 protein containing its amino acids positioning 1 to 377) was used for immunization as antigen. The DNA fragment corresponding to this PB1 region was amplified from pcDNA-PB1 by PCR with specific primers, PB1-FOR 2 and PB1-REV 2. The PCR products was phosphorylated with T4 polynucleotide kinase and digested with *Nde*I. The fragment was cloned into pET-14b between NdeI site and *Bam*HI site (*Bam*HI digested, and treated with Klenow fragment).
The His-PB2 fragment with a hexahistidine tag at its amino terminus (a mutant of PB2 protein containing its amino acids positioning 17 to 258) was used for immunization as antigen. The DNA fragment corresponding to this PB2 region was amplified from pcDNA-PB2 by PCR with specific primers, PB2-FOR 2 and PB2-REV 2. The PCR products were digested with *Xho*I, and cloned into *Xho*I-digested pET-14b plasmid.
Preparations of anti-PA and anti-NP antibodies were made as reported previously (Kawaguchi *et al*.; Takizawa *et al.*). These plasmids were used for transformation of *Escherichia coli* BL21 (DE3). Recombinant proteins recovered as insoluble fractions were solubilized with a guanidine buffer (20 mM Tris-HCl (pH 7.4), 6 M guanidine hydrochloride, and 500 mM NaCl). The solubilized recombinant proteins were purified using histidine tag-binding resin in the presence of 6 M guanidine hydrochloride according to the recommended protocol of pET System Manual (Novagen). The animals were boosted three times with 150 mg of each protein in Freund's incomplete adjuvant at 2-week intervals . Every antibody was used for immunological methods after appropriate dilution, so that the titer of each diluted antibody was exactly the same as the others in western blotting.

### - Preparation of recombinant proteins

The full-length *SUB2* gene was amplified by PCR using SUB2-FOR (His-) and SUB2-REV as primers and the yeast genomic DNA as template. The PCR products were phosphorylated with T4 polynucleotide kinase, and cloned into *Eco*RV-digested pBS. The resultant plasmid was designated pBS-His-SUB2.

To construct pET-21a-His-SUB2, the DNA fragment corresponding to the *SUB2* coding sequence was excised from pBS-His-SUB2 plasmid by digestion with NdeI and *Bam*HI. Then, this DNA fragment was cloned into NdeI- and *Bam*HI-digested pET-21a. This plasmid was used for transformation of *Escherichia coli* BL21 (DE3). The His-SUB2 protein was purified using histidine tag-binding resin. Preparation of His-RAF-2p48/UAP56 was made as reported previously (Momose *et al*.).

**[Table 1]**

| Table 1: Primers and oligonucleotides used in this study (Yeast genomic DNA and yeast genes) | | | |
|---|---|---|---|
| **Table S1. Primers and oligonucleotides used in this study.** | | | |
| **For yeast genomic DNA and yeast genes** | | | |
| **Primer name** | **Sequence (5' to 3')** | **Source** | **Nucleotide positions** |
| SUB2 up-FOR | GTTAGGTACCGAATACTTGGCCAAGATCTTGG | *SUB2* promoter (Chromosome IV) | 304495-304516^{a} |
| SUB2 up-REV | ACTTCCCGGGTCCAACAGGCAAACAGTTCCG | *SUB2* promoter | 305122-305141^{a} |
| SUB2 down-FOR | CCGTGAATTCAACGTAGTGTAGTCATTTCGTG | (Chromosome IV) *SUB2* terminator (Chromosome IV) | 306713-306734^{a} |
| SUB2 dwon-REV | CGTTGAATTCTTCTCCAACATCGATATCAGAG | *SUB2* terminator (Chromosome IV) | 307159-307180^{a} |
| ACT1-FOR | ATGGATTCTGAGGTTGCTGCTTTGGTTATT | *ACT1* cDNA | 1-30^{b} |
| ACT1-REV | GCGTGAGGTAGAGAGAAACC | *ACT1* cDNA | 502-521^{b} |
| ADH1-FOR | ATGTCTATCCCAGAAACTCAAAAAGG | *ADH1* cDNA | 1-26^{b} |
| ADH1-REV | AACGTTTTCACCCATGCCGACA | *ADH1* cDNA | 219-240^{b} |

**[Table 2]**

| (Continued Table 1) | | | |
|---|---|---|---|
| RDN25-1-FOR | CTGGTACCTTCGGTGCCC | *RDN25-1* cDNA | 125-142^{b} |
| RDN25-1-REV | CGCTATCGGTCTCTCGCCA | *RDN25-1* cDNA | 329-347^{b} |
| SUB2-FOR (His-) | TTTCATATGCATCATCATCATCATCATGGTTCACACGAAGGTGAAGAAGA TTTATT | *SUB2* cDNA | 4-29^{b} |
| SUB2 1062-FOR | GCTCGTTACAAGGCTTTCAAAGAT | *SUB2* cDNA | 1039-1062^{b} |
| SUB2-REV | CCGGATCCTTAATTATTCAAATAAGTGGACGGATCAATGCCTTC | *SUB2* cDNA | 1310-1341^{b} |
| TRP1-FOR | GGACGGCTAGCAACGACATTACTATATATATAATATAGGAAGC | *TRP1* cDNA | 1-32^{b} |
| TRP1-REV | GGCCGCGTCTCCCTTGGGCCCGGCAAGTGCACAAACAATACTTAAATA | *TRP1* cDNA | 977-1002^{b} |
| CYC1 TT-FOR | CCCCAAGCTTAGATCTGCGCCCGGGTCACCATCATGTAATTAGTTATGTC ACGCTTACATT | *CYC1* terminator | 1-28^{b} |
| CYC1 TT-REV | CGGACTAGTGATATCCCGGGGTCTAGAGCAAATTAAAGCCTTCGACGC | *CYC1* terminator | 692-720^{b} |

| | | | |
|---|---|---|---|
| a: Nucleotide positions correspond to those of yeast chromosome IV in the database (*Saccharomyces* Genome Database). *SUB2* is localized between nucleotide positions 305237 and 306577 of chromosome IV. b: The nucleotide position 1 is set to the 5'-terminus of each mRNA. In Table, "Primer name" indicates the name of primer, "Sequence" indicates sequence, and "Source" indicates gene forming a complementary strand with primer and oligonucleotide. | | | |

**[Table 3]**

| Table 2: Primers and oligonucleotides used in this study (Influenza viral genes) | | | |
|---|---|---|---|
| **For influenza viral genes** | | | |
| **Primer name** | **Sequence (5'to 3')** | **Source** | **Nucleotide positions** |
| PA-FOR | GATCCCGGGCATATGGAAGATTTTGTGCGACAATG | Segment 3 vRNA | 25-47^{c} |
| PA 664-FOR | ATCACAGGAACAATGCGCAAGC | Segment 3 vRNA | 643-664^{c} |
| PA 800-REV | GGTTCAATTCTAGCATTTACTTCTTT | Segment 3 vRNA | 775-800^{c} |
| PA3'-END | AGTAGAAACAAGGTACTTTTTTGGAC | Segment 3 cRNA | 2208-2233^{d} |
| PA 1356-FOR | CACATTGCAAGCATGAGAAGGAAT | Segment 3 c/mRNA | 1333-1356^{d} |
| PA 1683-REV | GGCACTTCTTAGAAGCATATCTC | Segment 3 c/mRNA | 1661-1683^{d} |
| NP-FOR | GGAATTCATATGGCGTCTCAAGGCACCAAACG | Segment 5 vRNA | 46-68^{e} |
| NP 444-FOR | GACGATGCAACGGCTGGTCTG | Segment 5 vRNA | 424-444^{e} |
| NP 614-REV | AGCATTGITCCAACTCCTTT | Segment 5 vRNA | 595-614^{e} |
| NP 3'-END | AGTAGAAACAAGGGTATTTTTCTTTA | Segment 5 cRNA | 1540-565^{f} |
| NP 846-FOR | CGGTCTGCACTCATATTGAGAGG | Segment 5 c/mRNA | 826-846^{f} |
| NP 1121-REV | GAAAGCTICCCTCITGGG | Segment 5 c/mRNA | 1104-1121^{f} |
| M1 3'-END | AGTAGAAACAAGGTAGTTTTTTACTC | Segment 7 cRNA | 26-43^{g} |

**[Table 4]**

| (Continued Table 2) | | | |
|---|---|---|---|
| M1 897-FOR | TAAATACGGACTGAAAGGAG | Segment 7 c/mRNA | 879-897^{g} |
| M1 1007-REV | TTACTCCAGCTCTATGCTGACAAAAT | Segment 7 c/mRNA | 982-1007^{g} |
| PB1-FOR 1 | CCCCAAGCTTGATATCGCGGCCGCCACCATGGATGTCAATCGACCTT | *PB1* cDNA | 1-20^{h} |
| PB1-REV 1 | CGCGTCGACGGTACCTATTTTTGCCGTCTGAGCTCTT | *PB1* cDNA | 2252-2274^{h} |
| PB2-FOR 1 | CGCGGATCCCGGGCGGCCGCCACCATGGAAAGAATAAAAGAACTAAG AAATCT | *PB2* cDNA | 1-29^{h} |
| PB2-REV 1 | CGCGCTCGAGCTAATTGATGGCCATCCGAATTC | *PB2* cDNA | 2258-2280^{h} |
| PA-FOR | CGCGGATCCGCGGCCGCCACCATGGAAGATTTTGTGCGACAATGCTTC | *PA* cDNA | 1-27^{h} |
| PA-REV | GCGGGGCCCTAACTCAATGCATGTGTAAGGAAGG | *PA* cDNA | 2126-2151^{h} |
| NP-FOR | TTGGATCCAAAATGGCTACTAAAGGTACTAAAAGATCT | *NP* cDNA | 1-27^{h} |
| NP-REV | GGAATTCATCTGATKTCGTACTCCTCTGCATTGT | *NP* cDNA | 1472-1497^{h} |
| PB1-FOR 2 | GATCCCGGGCATATGGATGTCAATCCGACCTTAC | *PB1* cDNA | 1-22^{h} |
| PB1-REV 2 | GCGCCTCGAGCTACTAATCGATGCTTGCTAGCATTTC | *PB1* cDNA | 1111-1132^{h} |
| PB2-FOR 2 | GCGCCTCGAGCGCGAGATACTCACAAAAACC | *PB2* cDNA | 49-69^{h} |
| PB2-REV 2 | GCGCCTCGAGCTACTAGCTTTGATCAACATCATCATT | *PB2* cDNA | 754-774^{h} |

| | | | |
|---|---|---|---|
| c, d, e, f and g: Nucleotide positions of influenza viral RNAs, where the nucleotide position 1 is set to the 5'-virus derived terminus of segment 3 vRNA^{c}, segment 3 cRNA and mRNA^{d}, segment 5 vRNA^{e}, segment 5 cRNA and mRNA^{f}, or segment 7 cRNA^{g}. h: The nucleotide position 1 is set to the 5'-terminus of each mRNA. In Table, "Primer name" indicates the name of primer, "Sequence" indicates sequence, and "Source" indicates gene forming a complementary strand with primer and oligonucleotide. | | | |

**[Table 5]**

| Table 3: Primers and oligonucleotides used in this study (Model viral genes and other oligonucleotides) | | | |
|---|---|---|---|
| **For model viral genes and other oligonucleotides** | | | |
| **Primer name** | **Sequence (5' to 3')** | **Source** | **Nucleotide positions** |
| Seg 5 digestion | GGAATTCATATGGTGATGGAATTGGTCAGAATGATCAAAC | Segment 5 vRNA | 616-640ⁱ |
| NS yEGFP-FOR | GTAGCAAAAGCAGGGTGACAAAGACATAATGTCTAAAGGTGAGAGATTA TTCACTG | *yEGFP* cDNA | 1-28^{k} |
| NS yEGFP-REV | AGTAGAAACAAGGGTGTTTTTT^{j}TTFATTTGTACAATTCATCCATACCATGG | *yEGFP* cDNA | 692-720^{k} |
| T7 vNS-FOR | TAATACGACTCACTAⁱAGTAGAAACAAGGGTGTTTT | Segment 8 vRNA | 1-20^{m} |
| vNS-REV | AGTAGAAACAAGGGTGTTTTTT | Segment 8 vRNA | 868-890^{m} |
| T7 cNS-FOR | TAATACGACTCACTATAⁱAGCAAAAGCAGGGTGACAAA | Segment 8 cRNA | 1-20ⁿ |
| cNS-REV | AGCAAAAGCAGGGTGACAAA | Segment 8 cRNA | 868-890ⁿ |
| 53-merVwt | AGUAGAAACAAGGGUGUUUUUCAUAUCAUUUAAACUUCACCCUGCU UUUGCU | Segment 8 vRNA | 1-22, 874-890° |

| | | | |
|---|---|---|---|
| i, m, n and ○: Nucleotide positions of influenza viral RNAs, where the nucleotide position 1 is set to the 5'-virus derived terminus of segment 5 vRNAⁱ, the 5'-terminus of segment 8 vRNA^{m and o}, and the 5'-terminus of segment 8 cRNAⁿ. j and l: Underlines indicate 3'- and 5'-terminal sequences of segment 8 cRNA^{j} and T7 promoter sequences^{l}. k: The nucleotide position 1 is set to the 5'-terminus of mRNA. In Table, "Primer name" indicates the name of primer, "Sequence" indicates sequence, and "Source" indicates gene forming a complementary strand with primer and oligonucleotide. | | | |

### <Literatures cited in Method 2>

Ito, H., Fukuda, Y., Murata, K. & Kimura, A. Transformation of intact yeast cells treated with alkali cations. J Bacteriol 153, 163-168 (1983).
Kawaguchi, A., Naito, T. & Nagata, K. Involvement of influenza virus PA subunit in assembly of functional RNA polymerase complexes. J Virol 79, 732-744 (2005).
Leeds, P., Peltz, S. W., Jacobson, A. & Culbertson, M. R. The product of the yeast UPF1 gene is required for rapid turnover of mRNAs containing a premature translational termination codon. Genes Dev 5, 2303-2314 (1991).
Lorenz, M. C. et al. Gene disruption with PCR products in Saccharomyces cerevisiae. Gene 158, 113-117 (1995).
Momose, F. et al. Cellular splicing factor RAF-2p48/NPI-5/BAT1/UAP56 interacts with the influenza virus nucleoprotein and enhances viral RNA synthesis. J Virol 75, 1899-1908 (2001).
Neumann, G. et al. Generation of influenza A viruses entirely from cloned cDNAs. Proc Natl Acad Sci U S A 96, 9345-9350 (1999).
Russell, P. J., Hambidge, S. J. & Kirkegaard, K. Direct introduction and transient expression of capped and non-capped RNA in Saccharomyces cerevisiae. Nucleic Acids Res 19, 4949-4953 (1991).
Takizawa, N. *et al.* Microbes and Infection in press.
Tu-ran, K. et al. Nuclear MxA proteins form a complex with influenza virus NP and inhibit the transcription of the engineered influenza virus genome. Nucleic Acids Res 32, 643-652 (2004).
Hereinbelow, the present invention will be described in detail based on Examples without limiting the scope of the invention.

### [Example 1]

### Introduction of vRNP into yeast cells

Budding yeast (*Saccharomyces cerevisiae*) strain YPH499 was used. The yeast was grown at 30°C in 50 ml of a complete medium (YPDA: yeast extract-peptone-dextrose medium containing adenine) until the cell density reached 3 x 10⁷ cells/ml. Then, the yeast cells were collected by centrifugation (at 800 x g for 2 minutes), and the supernatant was removed. The yeast cells were suspended in 20 ml of a 0.9% NaCl solution. The cells were collected by centrifugation (at 800 x g for 2 minutes), and the supernatant was removed. The yeast cells were suspended in 20 ml of a 1 M sorbitol solution. The yeast cells were collected by centrifugation (at 800 x g for 2 minutes), and the supernatant was removed. The yeast cells were suspended in 20 ml of a SCEM solution (1 M sorbitol, 0.1 M sodium citrate (pH 5.8), 10 mM EDTA, and 30 mM 2-mercaptoethanol). One hundred twenty-five units of Zymolyase-100T was added to the suspension containing the yeast cells, and the cells were incubated at 30°C for 45 to 60 minutes. After microscope investigation confirmed the breakage of cell walls of the yeast cells, the yeast cells were collected by centrifugation (at 400 x g for 1 minute), and the supernatant was removed. The yeast cells were suspended in 20 ml of a 1 M sorbitol solution. The yeast cells were collected by centrifugation (at 400 x g for 1 minute), and the supernatant was removed. The yeast cells were suspended in 20 ml of a STC solution (1 M sorbitol, 10 mM Tris-HCl (pH 7.4), and 10 mM CaCl₂). The yeast cells were collected by centrifugation (at 400 x g for 1 minute), and the supernatant was removed. The yeast cells were suspended in 2 ml of a STC solution. To 100 ml of the yeast suspension, 3 to 20 ml of vRNP was added. The mixture was gently stirred and was allowed to stand at room temperature for 10 minutes. Thereafter, 1 ml of a PEG solution (20% polyethylene glycol 6000, 10 mM Tris-HCl (pH 7.4), and 10 mM CaCl₂) was added. The mixture was gently stirred and was allowed to stand at room temperature for 10 minutes. The yeast cells were collected by centrifugation (at 400 x g for 1 minute), and the supernatant was removed. The yeast cells were suspended in 1 to 2 ml of a SOS solution (1 M sorbitol, 6.5 mM CaCl₂, and 50% YPDA) and were grown at 30°C.

### [Example 2]

### Replication and transcription of influenza viral genome in yeast cells

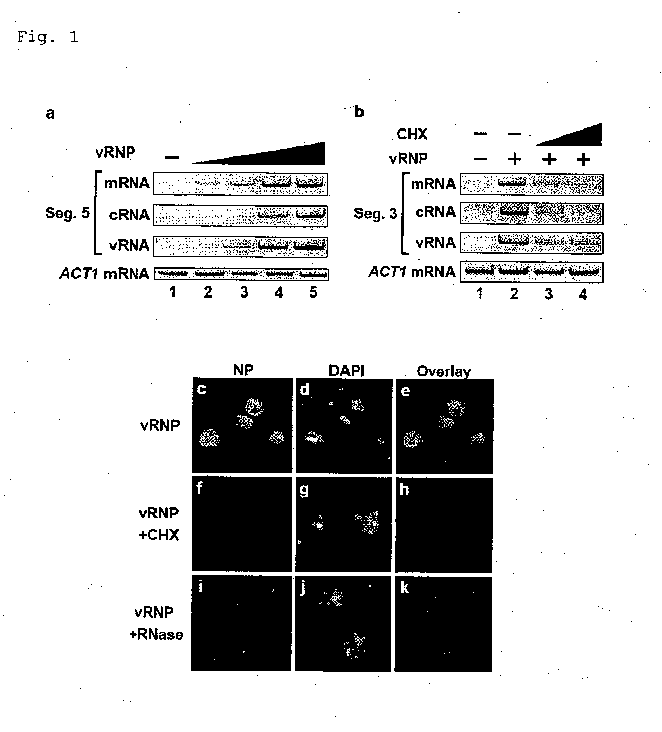

First, to examine whether or not vRNP purified from virions was infectious in yeast cells, vRNP was introduced into yeast cells as described above. The synthesis level of viral RNAs, *i.e.*., vRNA, cRNA (complementary RNA; the template for amplification of vRNA) and viral mRNA was analyzed by RT-PCR (Fig. 1a). The amount of viral RNAs synthesized in vRNP-transfected yeast cells increased depending on increasing amounts of transfected vRNP. This was also the case for other viral RNA segments (data notshown). These results demonstrate that the viral RNA polymerase and NP are active in yeast cells. In infected mammalian cells, the primary viral transcription is dependent on infecting vRNP, while the viral genome replication requires newly synthesized viral proteins⁴. When vRNP-transfected yeast cells were treated with cycloheximide (CHX), the synthesis level of viral RNAs was markedly reduced (Fig. 1b), indicating that the synthesis of viral RNAs in yeast was dependent on newly synthesized viral proteins. Since the viral mRNA synthesis is also sensitive to CHX treatment, most viral mRNA should be synthesized from newly synthesized vRNA as template in this system. Further, it could be concluded that viral mRNAs synthesized in yeast cells were functional. In indirect immunofluorescence assays (Fig. 1c-k), NP was found to be detected in vRNP-transfected yeast cells, but not in cells treated with CHX (Fig. 1f). When vRNP was treated with RNase A prior to transfection, the expression of NP was abolished (Fig. 1i), indicating that the viral gene expression was dependent on vRNA in vRNP complexes. Taken altogether, it is concluded that yeast cells support the viral genome replication and viral gene transcription depending on transfected vRNP complexes.

### (Description of Figure 1)

a: Yeast spheroplasts were mock-transfected (lane 1) or transfected with vRNP (transfected with vRNP containing 0.1, 0.3, 1 and 2 mg of NP for lanes 2, 3, 4 and 5, respectively) purified from virions. At 48 h post transfection (hpt), total yeast RNA was extracted and subjected to reverse transcription. PCR was then performed with primer sets specific for *ACT1* mRNA and negative-sense RNA (vRNA) of segment 5 and primer sets specific for positive-sense RNA (mRNA or cRNA). The amplified double-stranded DNAs were subjected to 7% polyacrylamide gel electrophoresis and visualized by ethidium bromide.
b: Yeast cells transfected with vRNP were incubated in the absence (lanes 1 and 2) or presence of 3 mg/ml (lane 3) and 10 mg/ml (lane 4) of cycloheximide (CHX). RT-PCR was performed with primers specific for segment 3 RNA and *ACT1* mRNA.
c-k: vRNP-transfected yeast cells were immunostained at 24 hpt. NP and DNA were stained with anti-NP antibody (c, f and i) and DAPI (d, g and j), respectively. Overlay: overlay of NP-stained and DAPI-stained panels (e, h and k). Yeast cells were incubated in the absence (c-e and i-k) or presence (f-h) of 5 mg/ml of CHX. Prior to transfection, vRNP was treated with RNase A for 10 minutes at 37°C (i-k).

### [Example 3]

### Complementation experiment of segment 5-depleted vRNP

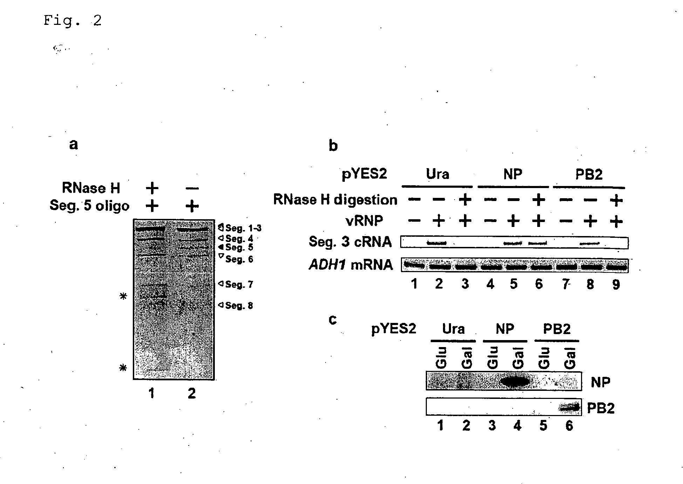

Complementation experiments were conducted using yeast cells expressing NP for vRNP complexes devoid of segment 5 vRNA that encoded NP. NP is required for formation of vRNP complexes and efficient elongation of the viral RNA chain. Segment 5 vRNA in vRNP complexes was eliminated with RNase H in the presence of an oligonucleotide complementary to a part of segment 5 vRNA¹² (see Method 2). By the RNase H treatment, the amount of segment 5 vRNA was significantly reduced, and some shorter bands appeared after digestion (Fig. 2a). Then, vRNP complexes lacking segment 5 vRNA (designated depleted vRNP) and mock-digested vRNP complexes were introduced into yeast cells. In the yeast cells transformed with pYES2 or pYES2-PB2, the depleted vRNP did not lead to the synthesis of cRNA (Fig. 2b, lanes 3 and 9). In contrast, the yeast cells transformed with pYES2-NP rescued the synthesis of cRNA (Fig. 2b, lane 6). Fig. 2c confirmed the expression of NP and PB2. These results indicate that NP expressed from plasmid complements the system using the depleted vRNP and consequently lacking the expression of NP. Further, it is found that the replication process in yeast cells is dependent on NP.

### (Description of Figure 2)

a: Digestion of segment 5 vRNA. vRNP (containing 3 mg of NP) was mixed with 300 ng of an oligonucleotide corresponding to a part of segment 5 vRNA (Method 1; Seg 5 digestion), in the presence of 0. 4 M NaCl for 5 minutes at 37 °C, and then treated (lane 1) or mock-treated (lane 2) with 30 U of RNase H for 5 minutes at 37 °C. The purified RNA was loaded onto a 3.2% polyacrylamide gel containing 7.7 M urea and was visualized by silver staining.

Asterisks indicate bands possibly corresponding to the digested fragments.
b: RT-PCR analysis of viral RNAs. vRNP and vRNP devoid of segment 5 vRNA (RNase H digestion) were transfected into yeast cells transformed with pYES2 (lanes 1-3), pYES2-NP (lanes 4-6) and pYES2-PB2 (lanes 7-9). The yeast cells were incubated for 24 hours in a medium containing galactose. RT-PCR analysis was performed with primer sets specific for segment 3 cRNA and *ADH1* mRNA.
c: Western blotting analysis of induced viral proteins. Control (lanes 1 and 2), NP (lanes 3 and 4), and PB2 (lanes 5 and 6).

### [Example 4]

### Replication and transcription of model viral RNA

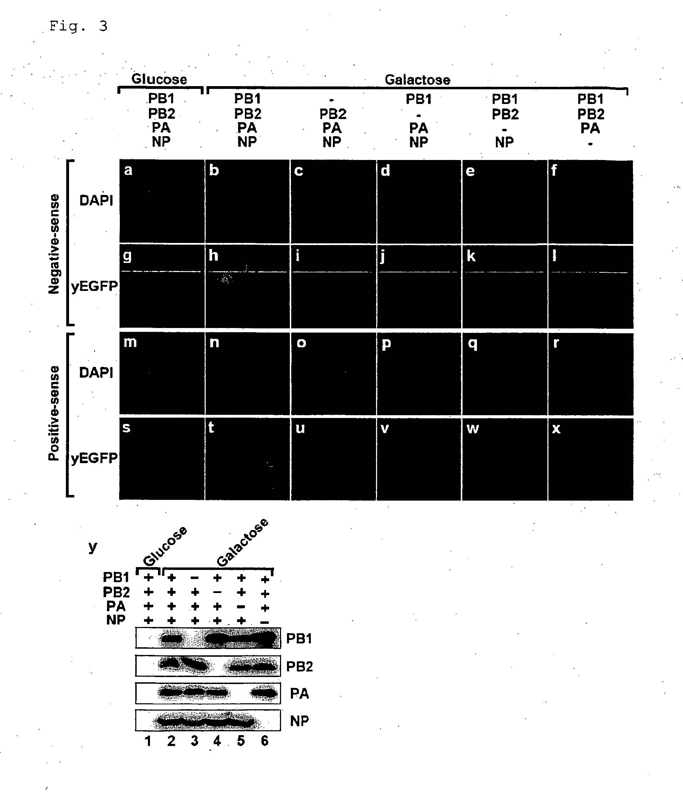

Next, attempts were made wherein all viral components constituting vRNP complexes were replaced with ones expressed from plasmids. Plasmids for expression of PB1, PB2, PA and NP under the control of the *GAL-1* promoter were constructed (see Method 2). After induction by galactose, viral proteins were synthesized in yeast cells transformed with these plasmids in combination (Fig. 3y). With use of the *in vitro* T7 RNA polymerase-directed transcription system^{1,2}, there were prepared both negative- and positive-sense model viral RNAs, *i.e.,* model vRNA and model cRNA (in which the yEGFP encoding region was sandwiched between the 5' - and 3' -terminal sequences of segment 8 vRNA encoding NS), designated vNS-yEGFP and vNS-yEGFP, respectively. Yeast cells expressing viral RNA polymerases and NP were transfected with the *in vitro* synthesized NS-yEGFP RNAs. The expression of yEGFP was detected from NS-yEGFP RNAs of both senses in yeast cells expressing galactose-induced three viral RNA polymerase subunits and NP (Fig. 3h and 3t). Five (5) to 10% of the yeast cells showed the detectable level of yEGFP (data not shown). In sharp contrast, yeast cells lacking either any one of the three polymerase subunits or NP did not support the expression of yEGFP (Fig. 3i-l and 3u-x).

These results indicate that the replication and transcription of the exogenously added model viral genome require three polymerase subunits and NP.

### (Description of Figure 3)

a-x: yEGFP expression from model viral RNA genomes containing yEGFP gene. Yeast spheroplasts expressing the viral RNA polymerase subunits (PB1, PB2 and PA) and NP were transfected with *in vitro* synthesized RNAs of vNS-yEGFP (negative-sense, a-l) or cNS-yEGFP (positive-sense, m-x), and were incubated in SD (glucose) or SG (galactose) medium at 30°C for 24 hours. DAPI-stained DNA (a-f and m-r) and yEGFP (g-l and s-x) were visualized under a fluorescent microscope.
y: Western blotting analysis of induced viral proteins. Yeast cells were transformed with plasmids as indicated at the top of the figure. Lysates prepared from exponentially growing transformants in either SD (lane 1) or SG (lanes 2-6) were subjected to the western blotting analysis.

### [Example 5]

### Effects of SUB2 deletion

Functions of RAF-2p48, a previously identified host factor¹³, on viral RNA synthesis were studied using the above system. RAF-2p48 facilitates formation of NP-RNA complexes and stimulates *in vitro* RNA synthesis from a model viral RNA. RAF-2p48 is identical with UAP56, an RNA splicing factor. SUB2, a putative *S. cerevisiae* orthologue of RAF-2p48/UAP56, associates with Sm snRNPs and is involved in their biogenesis *in vivo*¹⁴.

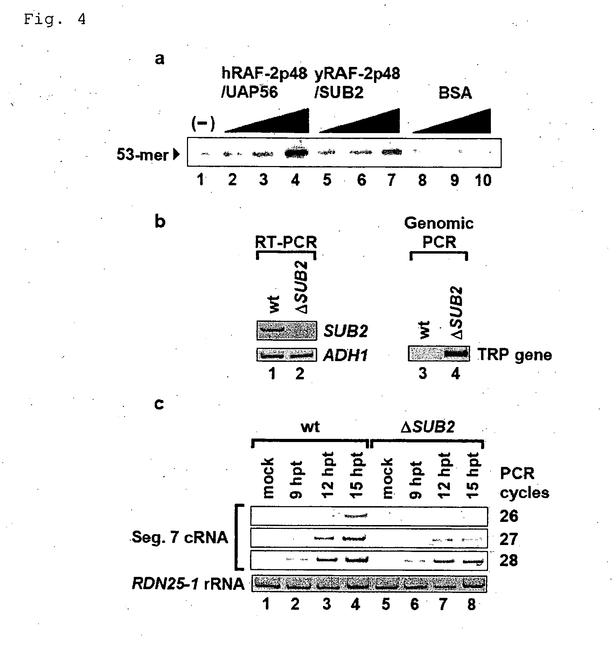

SUB2 shares 62% amino acid sequence identity with human RAF-2p48/UAP56¹⁵. Prior to genetical analyses of SUB2 in yeast cells, it was examined whether SUB2 stimulated the influenza virus RNA synthesis *in vitro.* Both recombinant RAF-2p48/UAP56 and SUB2 proteins stimulated *in vitro* RNA synthesis (Fig. 4a). The viral RNA synthesis stimulatory activity of SUB2 was approximately 50% that of RAF-2p48/UAP56. This result suggests that SUB2 may function as a host factor for the viral genome replication in yeast cells. Then, effects of the deletion of SUB2 on the viral RNA synthesis in yeast cells were examined.

*A SUB2* deletion strain was constructed by replacing its ORF with *TRP1* marker (Fig. 4b, right panel) (see Method 2), and was found to express no *SUB2* RNA (Fig. 4b, left panel). In the vRNP-transfected *SUB2* deletion strain, the rate of cRNA synthesis was markedly reduced compared with that in a wild type strain (Fig. 4c, compare with lanes 4 and 8 (PCR cycles: 26)). This was also the case for mRNA synthesis, although the effect of the deletion was greater for cRNA synthesis. This suggests that SUB2 facilitates the viral RNA synthesis as a host factor in yeast cells. After longer incubation of the vRNP-transfected yeast, the viral RNA synthesis activity in the SUB2 deletion strain resumed but to the less extent than that in wild type strain (Fig. 4c, compare with lanes 3 and 8). It is then assumed that yeast have another host factor(s) that complements the function of SUB2. On this line, the *SUB2* deletion strain is probably not lethal (data not shown).

Taken altogether, it is suggested that RAF-2p48/UAP56 is a host factor for the viral RNA synthesis in cells. The yeast system of the invention allows for the identification of more host factor candidates. Once a candidate is reached, one may go back to the cultured vertebrate cell system to verify and characterize the candidate.

### (Description of Figure 4)

a: The viral RNA synthesis stimulatory activity of recombinant RAF-2p48 and SUB2 proteins. *In vitro* RNA synthesis was carried out in the absence (lane 1) or presence of increasing amounts (10 ng (lanes 2, 5 and 8), 30 ng (lanes 3, 6 and 9) and 100 ng (lanes 4, 7 and 10)) of recombinant RAF-2p48 (lanes 2-4), SUB2 (lanes 5-7) or bovine serum albumin (BSA, lanes 8-10). The RNA product from the 53 base-long model viral genome is indicated by arrowhead.
b: RT-PCR and genomic PCR were carried out using total RNA and yeast genomic DNA (prepared from wild type strain and a *SUB2* deletion strain) with primer sets specific for *SUB2, ADH1* mRNA or *TRP1* gene.
c: Effects of SUB2 deletion on viral RNA synthesis. A wild type strain and SUB2 deletion strain were transfected with vRNP, and incubated for 9 hours (lanes 2 and 6), 12 hours (lanes 3 and 7), and 15 hours (lanes 4 and 8), and total RNA was extracted. RT-PCR was carried out with primer sets specific for segment 7 cRNA and RDN25-1 rRNA. The total RNA prepared from mock-transfected yeast cells was also analyzed (lanes 1 and 5).
In the above examples, the strains, materials and reagents only represent preferred embodiments of the present invention. The apparatuses, numerical conditions such as reagent concentrations, reagent amounts, treatment time and treatment temperatures, and treatment methods adopted in the above examples only represent preferred embodiments of the present invention.

### INDUSTRIAL APPLICABILITY

Some host factors involved in the influenza virus replication processes have been identified by biochemical methods^{13,16-19}. A systematic screening system has long been considered to be needed to further identify host factors and clarify their roles. The present invention discloses a novel system of the influenza virus genome replication and transcription using yeast cells. It has been shown that yeast cells support the replication of some positive-strand RNA viral (e.g., brome mosaic virus, tomato bushy stunt virus) genomes^{20,21}. These genetic systems have been proven to be useful for identification and characterization of host factors as well as viral factors. The newly developed system of the invention enables systematic and genetic screening of host factors. Since yeast genetics should be convenient and powerful, the strategy mimicking the present system is applicable for other negative-strand RNA viruses. Thus, it may be emphasized that the present yeast system may be applied for screening of anti-influenza virus drugs targeting viral factors and/or the interaction between viral factors and host factors.

### (Cited literature)

1. Luytjes, W., Krystal, M., Enami, M., Pavin, J. D. & Palese, P.. Amplification, expression, and packaging of foreign gene by influenza virus. Cell 59, 1107-1113 (1989).
2. Yamanaka, K., Ogasawara, N., Yoshikawa, H., Ishihama, A. & Nagata, K. In vivo analysis of the promoter structure of the influenza virus RNA genome using a transfection system with an engineered RNA. Proc Natl Acad Sci U S A 88, 5369-5373 (1991).
3. Neumann, G. et al. Generation of influenza A viruses entirely from cloned cDNAs. Proc Natl Acad Sci U S A 96, 9345-9350 (1999).
4. Kawaguchi, A., Naito, T. & Nagata, K. Involvement of influenza virus PA subunit in assembly of functional RNA polymerase complexes. J Virol 79, 732-744 (2005).
5. Taubenberger, J. K. et al. Characterization of the 1918 influenza virus polymerase genes. Nature 437, 889-893 (2005).
6. Ahlquist, P., Noueiry, A. O., Lee, W. M., Kushner, D. B. & Dye, B. T. Host factors in positive-strand RNA virus genome replication. J Virol 77, 8181-8186 (2003).
7. Prentice, E., McAuliffe, J., Lu, X., Subbarao, K. & Denison, M. R. Identification and characterization of severe acute respiratory syndrome coronavirus replicase proteins. J Virol 78, 9977-9986 (2004).
8. Scholle, F., Girard, Y. A., Zhao, Q., Higgs, S. & Mason, P. W. trans-Packaged West Nile virus-like particles: infectious properties in vitro and in infected mosquito vectors. J Virol 78, 11605-11614 (2004).
9. Lindenbach, B. D. et al. Complete replication of hepatitis C virus in cell culture. Science 309, 623-626 (2005).
10. Rochovansky, O. M. & Hirst, G. K. Infectivity and marker rescue activity of influenza virus ribonucleoprotein-polymerase complexes. Virology 73, 339-349 (1976).
11. Portela, A. & Digard, P. The influenza virus nucleoprotein: a multifunctional RNA-binding protein pivotal to virus replication. J Gen Virol 83, 723-734 (2002).
12. Enami, M. & Enami, K. Characterization of influenza virus NS1 protein by using a novel helper-virus-free reverse genetic system. J Virol 74, 5556-5561 (2000).
13. Momose, F. et al. Cellular splicing factor RAF-2p48/NPI-5/BAT1/UAP56 interacts with the influenza virus nucleoprotein and enhances viral RNA synthesis. J Virol 75, 1899-1908 (2001).
14. Noble, S. M. & Guthrie, C. Identification of novel genes required for yeast pre-mRNA splicing by means of cold-sensitive mutations. Genetics 143, 67-80 (1996).
15. Libri, D., Graziani, N., Saguez, C. & Boulay, J. Multiple roles for the yeast SUB2/yUAP56 gene in splicing. Genes Dev 15, 36-41 (2001).
16. Momose, F. et al. Identification of Hsp90 as a stimulatory host factor involved in influenza virus RNA synthesis. J Biol Chem 277, 45306-45314 (2002).
17. Wolff, T., O'Neill, R. E. & Palese, P. Interaction cloning of NS1-I, a human protein that binds to the nonstructural NS1 proteins of influenza A and B viruses. J Virol 70, 5363-5372 (1996).
18. Elton, D. et al. Interaction of the influenza virus nucleoprotein with the cellular CRM1-mediated nuclear export pathway. J Virol 75, 408-419 (2001).
19. Huarte, M., Sanz-Ezquerro, J. J., Roncal, F., Ortin, J. & Nieto, A. PA subunit from influenza virus polymerase complex interacts with a cellular protein with homology to a family of transcriptional activators. J Virol 75, 8597-8604 (2001).
20. Panavas, T., Serviene, E., Brasher, J. & Nagy, P. D. Yeast genome-wide screen reveals dissimilar sets of host genes affecting replication of RNA viruses. Proc Natl Acad Sci U S A 102, 7326-7331 (2005).
21. Kushner, D. B. et al. Systematic, genome-wide identification of host genes affecting replication of a positive-strand RNA virus. Proc Natl Acad Sci U S A 100, 15764-15769 (2003).
22. Spector, L. D. Cells: a laboratory manual, Cold Spring Harbor Laboratory, Cold Spring Harbor, N. Y, 106.3-106.7 (1998).
23. Yamanaka, K., Ishihama, A. & Nagata, K. Reconstitution of influenza virus RNA-nucleoprotein complexes structurally resembling native viral ribonucleoprotein cores. J Biol Chem 265, 11151-11155 (1990)

## Claims

1. A method for preparing a negative-strand RNA virus genome replication system in yeast, comprising introducing into yeast cells a viral RNA genome-viral protein complex (vRNP) purified from negative-strand RNA virus particles, and replicating and transcribing the negative-strand RNA genome in the yeast cells.

2. The method according to claim 1, wherein the negative-strand RNA virus is influenza virus.

3. The method according to claim 1 or 2, wherein the vRNP is introduced by being mixed with a yeast spheroplast in the presence of polyethylene glycol.

4. A method for preparing an influenza virus genome replication system, comprising introducing into yeast cells a model influenza viral RNA genome incorporated with a foreign reporter gene, and replicating and expressing the gene in the yeast cells.

5. A kit for preparing an influenza virus genome replication system, which kit comprises at least yeast cells, a purified influenza virus vRNP, reagents for preparing a yeast cell spheroplast, transfection reagents, and a vector for expressing influenza virus protein.

6. A method for characterizing viral factors of negative-strand RNA virus and screening host factors, wherein the method uses a negative-strand RNA virus genome replication system in yeast that is prepared by the foregoing method.

7. A method for screening host factors involved in the replication and transcription of influenza virus genome, wherein the method uses an influenza virus genome replication system in yeast that is prepared by the foregoing method.

8. A method for screening anti-influenza virus agents targeting at replication and transcription of influenza virus genome, wherein the method uses an influenza virus genome replication system in yeast that is prepared by the foregoing method.

9. A method for screening anti-virus agents targeting at interaction between a viral factor and a host factor.

10. A kit for measuring the expression level of a foreign reporter gene incorporated in an influenza viral RNA genome to determine functions of an anti-influenza virus candidate affecting the replication or transcription activity of influenza virus genome in yeast, the interaction between a viral factor and a host factor, or the virus growth-promoting effects of host factors.

11. A yeast transformant in which yeast is transformed by introduction of a negative-strand RNA.virus genome.
